# EUROPÄISCHE PATENTSCHRIFT

(11) **EP 0 720 614 B1**
(45) Veröffentlichungstag und Bekanntmachung des Hinweises auf die Patenterteilung: **24.05.2000**
(21) Anmeldenummer: 95928451.4
(22) Anmeldetag: 24.07.1995
(51) Int. Cl.: C07F 15/00, C12Q 1/68, G01N 33/58, C07F 19/00

(54) **METALLKOMPLEXE MIT GELADENEM LINKER**
METAL COMPLEXES WITH CHARGED LINKER
COMPLEXES METALLIQUES A LINKER CHARGE

(30) Priorität: 25.07.1994 DE 4426276; 31.08.1994 DE 4430998; 04.11.1994 DE 4439347; 04.11.1994 DE 4439345
(43) Veröffentlichungstag der Anmeldung: 10.07.1996
(73) Patentinhaber: Roche Diagnostics GmbH, 68305 Mannheim (DE)
(72) Erfinder: JOSEL, Hans-Peter, D-82362 Weilheim (DE); HÖSS, Eva, D-82319 Starnberg (DE); OFENLOCH-HÄHNLE, Beatus, D-82407 Wielenbach (DE); SEIDEL, Christoph, D-82362 Weilheim (DE); UPMEIER, Barbara, D-82393 Iffeldorf (DE); WIENHUES, Ursula-Henrike, D-82152 Krailling (DE)
(74) Vertreter: Weiss, Wolfgang, Dipl.-Chem. Dr.
(86) Internationale Anmeldenummer: EP9502920
(87) Internationale Veröffentlichungsnummer: WO9603409

(56) Entgegenhaltungen:
- WO-A-86/02734

## Beschreibung

Die vorliegende Erfindung betrifft neue Metallkomplexe mit geladenem Linker und deren Verwendung als lumineszierende Markierungsgruppen im Immunoassays.

Lumineszierende Metallkomplexe sind aus dem Stand der Technik bekannt. EP-A-0 178 450 offenbart Rutheniumkomplexe, die an ein immunologisch aktives Material gekoppelt sind, wobei die Rutheniumkomplexe drei gleiche oder verschiedene bi- oder polycyclische Liganden mit mindestens zwei stickstoffhaltigen Heterocyclen enthalten, wobei mindestens einer dieser Liganden mit mindestens einer wasserlöslich machenden Gruppe, wie -SO₃H oder -COOH substituiert ist, und wobei mindestens einer dieser Liganden mit mindestens einer reaktiven Gruppe wie -COOH direkt oder über eine Spacergruppe substituiert ist und wobei die Liganden über Stickstoffatome an das Ruthenium gebunden sind.

Die Einführung der kopplungsfähigen Gruppen erfolgt sehr umständlich durch Aktivierungs- und Folgereaktionen an den löslich machenden Gruppen der Liganden. Als besonders umständlich erweist sich auch die Herstellung monoaktivierter Verbindungen, die eine Kopplung mit biologischen Substanzen, z.B. Antikörpern ohne Vernetzung erlauben.

EP-A-0 580 979 offenbart die Verwendung von Osmium- oder Rutheniumkomplexen als Markierungsgruppen für die Elektrochemilumineszenz. Als Liganden für diese Komplexe werden stickstoffhaltige Heterocyclen, beispielsweise Bipyridine, genannt. WO 87/06706 offenbart weitere Metallkomplexe, die sich als Markierungsgruppen für Elektrochemilumineszenzmessungen eignen.

Weitere Nachteile der aus dem Stand der Technik bekannten Metallkomplexe bestehen in einer schlechten Quantenausbeute bei Elektrochemilumineszenzmessungen durch Sauerstoff-Quenching und Photodissoziation oder/und in einer hohen unspezifischen Bindung an Proteine.

Die der vorliegenden Erfindung zugrundeliegende Aufgabe bestand somit darin, die Nachteile des Standes der Technik mindestens teilweise zu beseitigen.

Überraschenderweise wurde festgestellt, daß die Einführung von freien positiven oder/und negativen Ladungsträgern in den Linker, der die reaktive kopplungsfähige Gruppe des Metallkomplexes mit einem der Liganden verbindet, die Adsorption von Konjugaten dieser Komplexe mit einer immunologisch reaktiven Substanz verringert und damit auch die Stabilität und Wiederfindung der Konjugate in Immunoassays verbessert. Überdies kann eine erhöhte Quantenausbeute erzielt werden.

Weiterhin wurde gefunden, daß lumineszierende Metallkomplexe mit geladenem Linker auf überraschend einfache Weise hergestellt werden können.

Ein Gegenstand der vorliegenden Erfindung ist somit ein Metallkomplex mit der allgemeinen Formel (I):

[M(L₁L₂L₃)]ₙ - Xₘ A (I)

worin M ein zwei- oder dreiwertiges Metallkation ausgewählt aus Seltenerde- oder Übergangmetallionen ist, L₁, L₂ und L₃ gleich oder verschieden sind und Liganden mit mindestens zwei stickstoffhaltigen Heterocyclen bedeuten, wobei L₁, L₂ und L₃ über Stickstoffatome an das Metallkation gebunden sind, X eine reaktive funktionelle Gruppe ist, die an mindestens einen der Liganden L₁, L₂, L₃ kovalent gebunden ist, n eine ganze Zahl von 1 bis 10 ist, m eine ganze Zahl von 1 bis 6 und vorzugsweise von 1 bis 3 ist und A die gegebenenfalls zum Ladungsausgleich erforderlichen Gegenionen bedeutet, wobei der Linker mindestens einen positiven oder/und negativen Ladungsträger enthält.

Der Metallkomplex ist vorzugsweise ein lumineszierender Metallkomplex, d.h. ein Metallkomplex, der eine nachweisbare Lumineszenzreaktion erzeugen kann. Der Nachweis dieser Lumineszenzreaktion kann beispielsweise durch Fluoreszenz- oder durch Elektrochemilumineszenzmessung erfolgen. Das Metallkation in diesem Komplex ist beispielsweise ein Übergangsmetall oder ein Seltenerdenmetall. Vorzugsweise ist das Metall Ruthenium, Osmium, Rhenium, Iridium, Rhodium, Platin, Indium, Palladium, Molybdän, Techneticum, Kupfer, Chrom oder Wolfram. Besonders bevorzugt sind Ruthenium, Iridium, Rhenium, Chrom und Osmium. Am meisten bevorzugt ist Ruthenium.

Die Liganden L₁, L₂ und L₃ sind Liganden mit mindestens zwei stickstoffhaltigen Heterocyclen. Bevorzugt sind aromatische Heterocyclen wie z.B. Bipyridyl, Bipyrazyl, Terpyridyl und Phenanthrolyl. Besonders bevorzugt werden die Liganden L₁, L₂ und L₃ aus Bipyridin- und Phenanthrolin-Ringsystemen ausgewählt.

Die reaktive funktionelle Gruppe X des Komplexes ist eine reaktive Gruppe, die mit einer immunologisch aktiven Substanz gekoppelt werden kann. Vorzugsweise ist die Gruppe X eine aktivierte Carbonsäuregruppe wie etwa ein Carbonsäurehalogenid, ein Carbonsäureanhydrid oder ein Aktivester, z.B. ein N-Hydroxysuccinimid-, ein p-Nitrophenyl-, Pentafluorphenyl-, Imidazolyl- oder N-Hydroxybenzotriazolylester, ein Maleimid oder eine photoaktivierbare Gruppe, z.B. ein Azid. Vorzugsweise enthält der Komplex nur eine einzige funktionelle Gruppe X.

Weiterhin kann der Komplex gegebenenfalls eine oder mehrere zum Ladungsausgleich erforderliche Gegenionen A enthalten. Beispiele für geeignete negativ geladene Gegenionen sind Halogenide, OH⁻, Carbonat, Alkylcarboxylat, z. B. Trifluoracetat, Sulfat, Hexafluorophosphat- und Tetrafluoroborat-Gruppen. Hexafluorophosphat-, Trifluoracetat- und Tetrafluoroborat-Gruppen sind besonders bevorzugt. Beispiele für geeignete positiv geladene Gegenionen sind monovalente Kationen wie etwa Alkalimetall- und Ammoniumionen.

Der erfindungsgemäße Metallkomplex unterscheidet sich von den aus dem Stand der Technik bekannten Metallkomplexen dadurch, daß er im Linker zwischen dem Liganden und der reaktiven kopplungsfähigen Gruppe X mindestens einen Ladungsträger enthält. Der Begriff "Ladungsträger" bedeutet im Sinne der vorliegenden Erfindung eine Gruppe, die bei einem pH-Wert im Bereich von 6 - 8 überwiegend in ionischer Form vorliegt. Der Linker enthält vorzugsweise bis zu 10, besonders bevorzugt 2-8 und am meisten bevorzugt 2-4 solcher Ladungsträger.

Besonders bevorzugt enthält der Linker mindestens einen negativen Ladungsträger. Beispiele für geeignete negative Ladungsträger sind Phosphat-, Phosphonat-, Sulfonat- und Carboxylatgruppen, wobei Carboxylatgruppen am meisten bevorzugt sind.

Beispiele für positive Ladungsträger sind Amino- und ein- oder mehrfach substituierte Aminogruppen, wie etwa Mono-, Di- oder Trialkylaminogruppen, wobei Alkyl einen geraden oder verzweigten Alkylrest von 1-6 C-Atomen oder einen zyklischen Alkylrest von 3-6 C-Atomen bedeutet. Besonders bevorzugt werden die positiven Ladungsträger aus basischen Aminosäuren wie etwa Lysin oder substituierten Aminosäuren wie etwa Diethyllysin ausgewählt. Amine und substituierte Amine können auch als Elektronendonoren beim Nachweis der Metallkomplexe durch Elektrochemilumineszenz dienen.

Der Linker hat eine Kettenlänge von vorzugsweise 4-40 Atomen und ist eine durch Einbau von Heteroatomen, z.B. Amidfunktionen, modifizierte Alkylenkette. Die Ladungsträger sind im Linker vorzugsweise derart angeordnet, daß ein H-Atom einer Alkyleneinheit durch eine geladene Gruppe, z.B. NH₂⁺ oder C0₂⁻ersetzt ist.

Vorzugsweise ist der Linker, der die freien Ladungsträger enthält, zumindest teilweise aus Aminocarbonsäure-Einheiten aufgebaut, die über Peptidbindungen miteinander verknüpft sind. Bei einem derartigen Linker können die Ladungsträger aus freien Amino- oder/und Carboxylatgruppen von polyfunktionellen Aminocarbonsäuren stammen, die insgesamt mindestens 3 geladene Gruppen (Amino- plus Carboxylat) enthalten, so daß nach Einbau in den Linker und damit verbundener Reaktion von 2 der geladenen Gruppen noch mindestens ein freier Ladungsträger vorhanden ist. Beispielsweise können die Ladungsträger von trifunktionellen Aminocarbonsäuren stammen, die (a) eine Aminogruppe und 2 Carboxylatgruppen oder (b) 2 Aminogruppen und eine Carboxylatgruppe enthalten. Beispiele für derartige trifunktionelle Aminocarbonsäuren sind Lysin, Ornithin, Hydroxylysin, Asparaginsäure und Glutaminsäure.

Der erfindungsgemäße Metallkomplex kann weiterhin mindestens eine hydrophile Gruppe ausgewählt aus C₂-C₃-Alkylenoxy-Einheiten, C₂-C₃-Alkylenthio-Einheiten, C₂-C₃-Alkylenamino-Einheiten und Polyhydroxy-Einheiten enthalten.

Die Polyhydroxy-Einheiten werden vorzugsweise aus Gruppen der Formeln (IIa) oder (IIb) ausgewählt:

-NR-W (IIa)

-O-W (IIb)

worin W einen organischen Rest mit mindestens zwei Hydroxygruppen und R Wasserstoff oder C₁-C₅-Alkyl bedeutet. Der organische Rest W enthält vorzugsweise 2 bis 6 und besonders bevorzugt 2 bis 4 Hydroxygruppen. Weiterhin sollte W günstigerweise 2 bis 10 und insbesondere 3-6 Kohlenstoffatome enthalten. Spezifische Beispiele für geeignete Polyhydroxy-Einheiten sind Reste von Polyalkoholen wie etwa Glycerin oder Aminopolyalkoholen. Ein bevorzugter Aminopolyalkohol ist Tris (2-Amino-2-(hydroxymethyl)-1,3-propantriol). In diesem Fall weist die Polyhydroxy-Einheit die Formel NH-C(CH₂OH)₃ auf. Die Polyalkohole bzw. Aminopolyalkohole sind an dem Metallkomplex vorzugsweise in Form von Estern bzw. Amiden gekoppelt. Die OH-Gruppen der Polyalkohole bzw. Aminopolyalkohole können gegebenenfalls durch hydrophile Gruppen, z.B. durch dendrimere Gruppen substituiert sein.

Die C₂-C₃-Alkylenoxy-, C₂-C₃-Alkylenthio- und C₂-C₃-Alkylenamino-Einheiten des erfindungsgemäßen Metallkomplexes sind vorzugsweise C₂-Einheiten und insbesondere Ethylenoxy-Einheiten. Der Komplex enthält pro Metallkation vorzugsweise 1 bis 30 und besonders bevorzugt 2 bis 20 C₂-C₃-Alkylenoxy-, C₂-C₃-Alkylenthio- bzw. C₂-C₃-Alkylenamino-Einheiten. Diese Einheiten sind Bestandteile von Substituenten der heterocyclischen Liganden des Metallkomplexes. Sie können im Linker zwischen einem der Liganden und der reaktiven funktionellen Gruppe X oder/und in einfachen Substituenten vorliegen. Die Alkylenoxy-, Alkylenthio-bzw. Alkylenamino-Einheiten können auch über einen Brückenkopf miteinander verknüpft sein, der gegebenenfalls eine funktionelle Gruppe X tragen kann. Andererseits können über den Brückenkopf auch mehrere Komplex-Einheiten miteinander verknüpft sein.

In einer Ausführungsform der vorliegenden Erfindung besitzt der erfindungsgemäße Metallkomplex die allgemeine Formel (III): 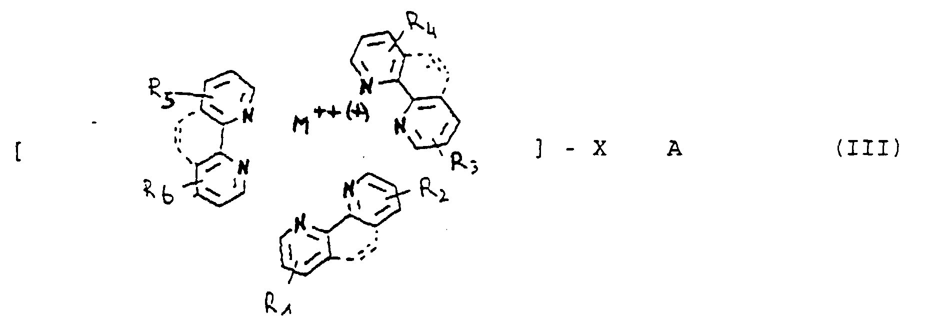 worin M, X und A wie vorstehend definiert sind, R₁, R₂, R₃, R₄, R₅ und R₆ gleich oder verschieden sind und jeweils einen oder mehrere Substituenten bedeuten, unter der Voraussetzung, daß X über einen der Substituenten R₁, R₂, R₃, R₄, R₅ oder R₆ als Linker an einen der Liganden gebunden ist.

Die Liganden des Komplexes sind je nach Anwesenheit bzw. Abwesenheit der durch gebrochene Linien bezeichneten Gruppen gegebenenfalls substituierte Phenanthrolin- bzw. Bipyridinsysteme.

Die Substituenten R₁, R₂, R₃, R₄, R₅ und R₆ an den Liganden sind - sofern sie nicht den Linker der Gruppe X enthalten - vorzugsweise Wasserstoff, C₁-C₅-Alkyl, insbesondere C₁-C₃-Alkyl, oder eine hydrophile Gruppe wie oben definiert.

In einer besonders bevorzugten Ausführungsform besitzen die Metallkomplexe die allgemeine Formel (IIIa): 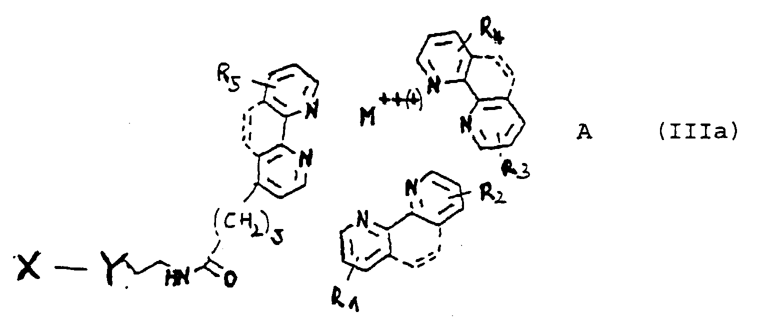 worin M, X und A wie vorstehend definiert sind, R₁, R₂, R₃, R₄ und R₅ wie vorstehend definiert sind, s eine ganze Zahl von 0 bis 6 vorzugsweise von 1 bis 4 ist und Y den Linker mit freien Ladungsträgern bedeutet.

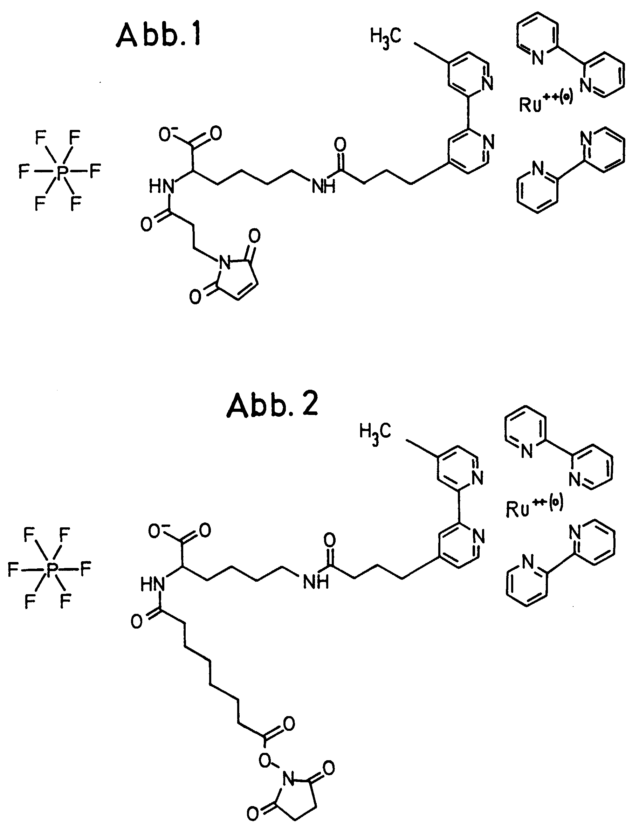

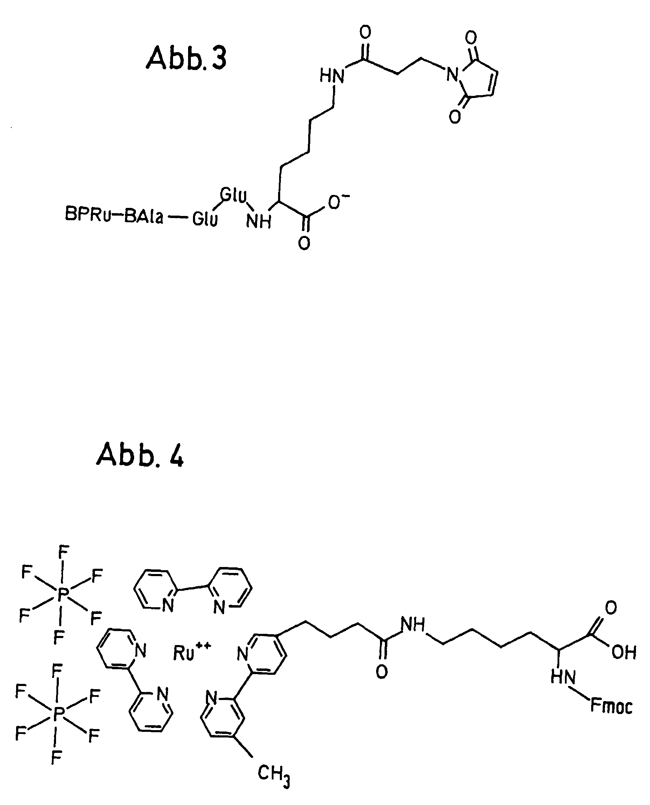

Beispiele für Verbindungen der Formeln (III) bzw. (IIIa) sind in Abb. 1-3 dargestellt. Abb. 1 und 2 zeigen Komplexe, die einen Linker mit jeweils einem freien negativen Ladungsträger aufweisen. Der Linker enthält jeweils eine trifunktionelle Aminosäure, nämlich Lysin, deren Aminogruppen zur Ausbildung von Peptidbindungen im Linker dienen, während die Carboxylatgruppe den freien Ladungsträger bildet. Abb. 3 zeigt einen Linker, aus 4 Aminosäure-Einheiten, nämlich β-Alanin, Lysin und zweimal Glutaminsäure aufgebaut ist. Der Linker enthält 3 negative Ladungsträger, jeweils eine Carboxylatgruppe von den beiden Glu-Resten und eine Carboxylatgruppe, die von Lys stammt. Die funktionelle Gruppe X ist in Abb. 1 und 3 ein zur Kopplung an SH-Gruppen geeignetes Maleimid und in Abb. 2 ein zur Kopplung an NH₂-Gruppen geeigneter N-Hydroxysuccinimidester.

Die Liganden des erfindungsgemäßen Metallkomplexes können auch miteinander verknüpft sein, so daß der Metallkomplex in Form eines Halbkäfigs bzw. Käfigs vorliegt. Eine bevorzugte Ausführungsform eines erfindungsgemäßen Metallkomplexes in Form eines Halbkäfigs oder Käfigs besitzt die allgemeine Formel (IV): 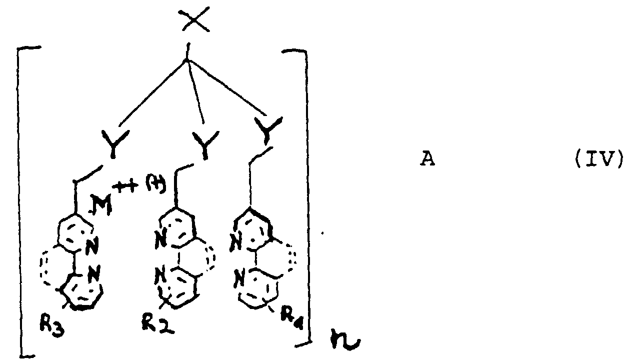 worin M, X, n und A wie vorstehend definiert sind, R₁, R₂ und R₃ gleich oder verschieden sind und jeweils einen oder mehrere Substituenten - wie vorstehend definiert - an dem Bipyridin- oder Phenanthrolin-Liganden bedeuten und Y jeweils einen Linker mit mindestens einem Ladungsträger bedeutet.

Wenn die Substituenten R₁, R₂ und R₃ in Formel (IV) und gegebenenfalls über Linkergruppen kovalent miteinander verknüpft sind, dann besitzt der Komplex der Formel (IV) die Form eines Käfigs.

Der Komplex der Formel (IV) kann nicht nur als Monomer, sondern als Oligomer aus vorzugsweise bis zu 5 einzelnen Metallkomplexen vorliegen. Hierzu kann die funktionelle kopplungsfähige Gruppe X beispielsweise ein Substituent an einem aromatischen Kern, z.B. einem Phenylkern sein, wobei zwei oder mehr der restlichen Substituentenpositionen des aromatischen Kerns durch einen halbkäfig- bzw. käfigförmigen Metallkomplex substituiert sein können.

Die Herstellung der erfindungsgemäßen Metallkomplexe kann durch Reaktion eines Metallsalzes, z.B. eines Metallhalogenids mit den entsprechenden Liganden und ggf. anschließenden Austausch des Halogenidions durch Hexafluorophosphat-, Trifluoracetat- oder Tetrafluoroborat-Anionen erfolgen. Derartige Verfahren sind im Stand der Technik, z.B. in EP-B-0 178 450 und EP-B-0 255 534 beschrieben. Auf diese Offenbarung wird hiermit Bezug genommen.

Der Aufbau eines geladenen Linkers an einem N-heterocyclischen Liganden mit einem geladenen Linker kann einerseits als Kopplungsreaktion in Lösung durchgeführt werden, wobei eine gegebenenfalls partiell geschützte Aminocarbonsäure an eine reaktive Gruppe des Liganden, z.B. eine Carbonsäure, gekoppelt wird. Dieser Kopplungsabschnitt kann gegebenenfalls nochmals wiederholt werden, bis ein Linker mit der gewünschten Länge aufgebaut ist. Dabei wird mindestens eine polyfunktionelle Aminocarbonsäure eingeführt, die eine oder mehrere geladene Seitengruppen enthält.

Anschließend wird die reaktive Gruppe X eingeführt und gegebenenfalls vorhandene Schutzgruppen auf den Seitengruppen der Aminocarbonsäuren werden abgespalten. Dieser Aufbau des Liganden durch sukzessive Ankopplung von Aminosäuren in Lösung kann einerseits an einem einzelnen Liganden und andererseits an einem bereits an einen Metallkomplex gebundenen Liganden als Ausgangsmaterial erfolgen. Ein geeignetes Ausgangsmaterial ist z.B. ein lumineszierender Metallkomplex, der eine freie Carboxylatgruppe enthält. Derartige Komplexe sind aus den obengenannten Dokumenten bekannt und werden auch z.B. von der Firma Igen Inc., Rockville, MD, USA kommerziell angeboten.

Andererseits kann die Herstellung der Komplexe auch durch Festphasen-Peptidsynthese erfolgen. In einer ersten Ausführungsform der Festphasensynthese wird eine Aminosäure über ihre Carboxylatgruppe an den Festphasenträger gekoppelt und dann durch sukzessive Kopplung weiterer Aminosäuren der gewünschte Linker aufgebaut. Zur Herstellung eines erfindungsgemäßen Linkers wird dabei mindestens eine Aminosäure verwendet, die als Seitengruppe eine geladene Gruppe, z.B. eine Amino- oder eine Carboxylatgruppe, gegebenenfalls in geschützter Form enthält. Nach Fertigstellung der gewünschten Linkersequenz kann ein aktivierter Metallkomplex, z.B. als Aktivester, an die freie N-terminale Aminogruppe des festphasengebundenen Peptids gekoppelt werden. Nach Abspaltung von der Festphase kann die reaktive Gruppe X an den Carboxyterminus des Peptidlinkers gekoppelt und gegebenenfalls vorhandene Schutzgruppen abgespalten werden.

In einer anderen Ausführungsform der Festphasensynthese kann ein Aminosäure-Metallkomplex-Konjugat, das eine geschützte Aminogruppe und eine Carboxylatgruppe enthält, z.B. Fmoc-Lys (BRu)-OH (Abb. 4) über die freie Carboxylatgruppe an eine Festphase verankert werden und nach Freisetzung der blockierten Aminogruppe ein Peptidlinker aufgebaut werden. Nach Fertigstellung der gewünschten Linkersequenz wird der Komplex von der Festphase abgespalten, wobei ein Linker erhalten wird, der zumindest die ursprüngliche Carboxylatankergruppe als freien Ladungsträger enthält. Die reaktive Gruppe X kann an den Aminoterminus des resultierenden Peptidlinkers gekoppelt werden.

In einer dritten Ausführungsform der Festphasensynthese kann die Linkersequenz mit Ladungsträgern auch direkt an ein ausgewähltes Peptidepitop synthetisiert werden.

Zur Herstellung der erfindungsgemäßen Metallkomplexe kann auch eine Kombination der obengenannten Synthesevarianten erfolgen. Aminosäure-Metallkomplex-Konjugate, die zur Festphasensynthese der erfindungsgemäßen Komplexe mit geladenem Linker geeignet sind, werden in DE-A-44 30 998.8 beschrieben. Auf diese Offenbarung wird hiermit Bezug genommen.

Die Herstellung von Metallkomplexen der Formel (IV) mit Halbkäfig- oder Käfigstruktur kann beispielsweise erfolgen durch Anfügen von geladenen Linkern an die Bipyridin- oder Phenanthrolinliganden und Knüpfung dieser Einheiten an einen Brückenkopf über eine Amidbindung. Bei Verwendung von zwei Brückenköpfen können vollständige Käfigstrukturen erhalten werden. Bevorzugt ist die Knüpfung von drei Liganden an einen trivalenten Brückenkopf, z.B. Tris.

Ein weiterer Gegenstand der vorliegenden Erfindung ist ein Konjugat, umfassend eine biologische Substanz, an die mindestens ein erfindungsgemäßer Metallkomplex gekoppelt ist. Beispiele für geeignete biologische Substanzen sind Zellen, Viren, subzelluläre Teilchen, Proteine, Lipoproteine, Glycoproteine, Peptide, Polypeptide, Nukleinsäuren, peptidische Nukleinsäuren (PNA), Oligosaccharide, Polysaccharide, Lipopolysaccharide, zelluläre Metaboliten, Haptene, Hormone, pharmakologische Wirkstoffe, Alkaloide, Steroide, Vitamine, Aminosäuren und Zucker.

Die Kopplung des Metallkomplexes mit der biologischen Substanz erfolgt über die reaktive funktionelle Gruppe des Metallkomplexes, die mit einer funktionellen Gruppe der biologischen Substanz kovalent kuppeln kann. Wenn die funktionelle Gruppe ein Aktivester ist, kann beispielweise eine Kopplung mit freien Aminogruppen der biologischen Substanz erfolgen. Wenn die funktionelle Gruppe ein Maleimidrest ist, kann eine Kopplung mit freien SH-Gruppen der biologischen Substanz erfolgen.

Bei einer besonders bevorzugten Ausführungsform der vorliegenden Erfindung werden die Metallkomplexe an ein Peptid gekoppelt, das vorzugsweise eine Länge von maximal 50 Aminosäuren und besonders bevorzugt von maximal 30 Aminosäuren aufweist. Die Herstellung dieser Metallkomplex-markierten Peptide erfolgt vorzugsweise dadurch, daß man ein Peptid mit der gewünschten Aminosäuresequenz an einer Festphase synthetisiert, wobei man a) nach der Synthese einen aktivierten Metallkomplex, vorzugsweise ein Metallkomplex-Aktivesterderivat an die N-terminale Aminogruppe des Peptids koppelt oder/und b) während der Synthese an mindestens einer Position des Peptids ein Aminosäurederivat einführt, das kovalent mit einem Metallkomplex gekoppelt ist. Die Kopplung des Metallkomplexes an die N-terminale Aminosäure des Peptids erfolgt vorzugsweise vor Abspaltung des Peptids von der Festphase und vor einer Abspaltung von Schutzgruppen an reaktiven Seitengruppen der zur Peptidsynthese verwendeten Aminosäurederivate.

Die Peptide enthalten vorzugsweise einen immunologisch reaktiven Epitopbereich und einen Spacerbereich, wobei mindesens eine Metallkomplex-Markierungsgruppe an den Spacerbereich gekoppelt wird. Der Spacerbereich weist vorzugsweise eine Länge von 1 bis 10 Aminosäuren auf und ist am Amino- oder/und Carboxyterminus des Peptids angeordnet.

Der Spacerbereich enthält vorzugsweise Aminosäuren, die Ladungen aufweisen oder/und Wasserstoffbrücken ausbilden können. Die Aminosäuren des Spacerbereichs werden vorzugsweise gebildet aus der Gruppe bestehend aus Glycin, β-Alanin, γ-Aminobuttersäure, ε-Aminocapronsäure, Lysin und Verbindungen der Strukturformel NH₂-[(CH₂)_{y}O]ₓ-CH₂-CH₂-COOH, worin y 2 oder 3 ist und x 1 bis 10 ist.

Die Epitopbereiche der Peptide stammen vorzugsweise aus pathogenen Organismen, z.B. Bakterien, Viren, und Protozoen, oder aus Autoimmun-Antigenen. Besonders bevorzugt stammt der Eptitopbereich aus viralen Antigenen, z.B. den Aminosäuresequenzen von HIVI, HIVII oder Hepatitis C-Virus (HCV).

Weitere bevorzugte Beispiele für biologische Substanzen sind Biotin, Nukleinsäuren, Antikörper oder Antikörperfragmente, Polypeptidantigene, d.h. immunologisch reaktive Polypeptide, oder Haptene, d.h. organische Moleküle mit einem Molkulargewicht von 150 bis 2000, insbesondere Moleküle mit einem Steroidgrundgerüst, wie etwa Cardenolide, Cardenolid-Glycoside (z.B. Digoxin, Digoxigenin), Steroid-Alkaloide, Sexual-hormone (z.B. Progesteron), Glucocorticoide etc. Weitere Beispiele für Haptene sind Prostaglandine, Leucotriene, Leuco-en-diine, Thromboxane etc.

Noch ein weiterer Gegenstand der vorliegenden Erfindung ist die Verwendung der erfindungsgemäßen Metallkomplexe, bzw. der erfindungsgemäßen Konjugate in einem immunologischen Nachweisverfahren oder einem Nukleinsäure-Hybridisierungsverfahren, insbesondere in einem Lumineszenz-Assay.

Dabei wird der Metallkomplex als Markierungegruppe verwendet, mit deren Hilfe die qualitative oder/und quantitative Bestimmung eines Analyten in einer Probelösung möglich ist. Der Nachweis des Metallkomplexes erfolgt vorzugsweise durch Elektrochemilumineszenz, wobei lumineszierende Spezies elektrochemisch an der Oberfläche einer Elektrode erzeugt werden. Beispiele zur Durchführung von Lumineszenz-Assays mit Metallkomplexen des Standes der Technik finden sich in EP-A-0 580 979, WO 90/05301, WO 90/11511 und WO 92/14138. Auf die dort offenbarten Verfahren und Vorrichtungen für Lumineszenz-Assays wird hiermit Bezug genommen. Die Elektrochemilumineszenz-Assays werden in Gegenwart einer Festphase durchgeführt, die vorzugsweise aus Mikropartikeln, insbesondere aus magnetischen Mikropartikeln besteht, die mit einer reaktiven Beschichtung versehen sind, z.B. mit Streptavidin. Auf diese Weise können Immun- oder Hybridisierungskomplexe , die einen Metallkomplex als Markierungsgruppe enthalten, an die Festphase gebunden nachgewiesen werden.

Die Elektrochemilumineszenz-Messung wird vorzugsweise in Gegenwart eines Reduktionsmittels für den Metallkomplex durchgeführt, z.B. einem Amin. Bevorzugt sind aliphatische Amine, insbesondere primäre, sekundäre und tertiäre Alkylamine, deren Alkylgruppen jeweils 1 bis 3 Kohlenstoffatome aufweisen. Besonders bevorzugt ist Tripropylamin. Das Amin kann jedoch auch ein aromatisches Amin, wie Anilin oder ein heterocyclisches Amin sein. Das Reduktionsmittel kann bereits in der Ligandensphäre des Komplexes integriert sein.

Weiterhin kann ggf. Verstärker ein nichtionisches oberflächenaktives Mittel, z.B. ein ethoxyliertes Phenol vorhanden sein. Derartige Substanzen sind beispielsweise kommerziell unter den Bezeichnungen Triton X100 oder Triton N401 erhältlich.

Andererseits kann der Nachweis des lumineszierenden Metallkomplexes auch durch Fluoreszenz erfolgen, wobei das Metallchelat durch Bestrahlung mit einem Licht der geeigneten Wellenlänge angeregt und die daraus resultierende Fluoreszenzstrahlung gemessen wird. Beispiele zur Durchführung von Fluoreszenz-Assays finden sich in EP-A-0 178 450 und EP-A-0 255 534. Auf diese Offenbarung wird hiermit Bezug genommen.

Das zuvor beschriebene Prinzip der Verwendung von Metallkomplexen mit geladenen Linkern kann im übrigen auch auf andere Markierungs- oder/und Festphasenbindungsgruppen übertragen werden. Auf diese Weise erhält man Markierungs- oder/und Festphasenbindungsgruppen mit einer kovalent über einen Linker gebundenen, reaktiven funktionellen Gruppe X, wobei der Linker mindestens einen positiven oder/und negativen Ladungsträger enthält. Vorzugsweise ist der Linker zumindest teilweise aus Aminocarbonsäure-Einheiten aufgebaut, die über Peptidbindungen miteinander verknüpft sind.

Die Markierungs- oder/und Festphasenbindungsgruppen sind vorzugsweise ausgewählt aus Fluoreszenzmarkierungsgruppen, z.B. Fluorescein, Cumarin, Rhodamin, Resorufin, Cyanin und Derivaten davon, sowie aus Biotin und Biotinanaloga, wie etwa Iminobiotin oder Desthiobiotin.

Es lassen sich auch Konjugate der zuvor genannten Markierungs- oder/und Festphasenbindungsgruppen mit einer biologischen Substanz wie zuvor definiert herstellen. Die Markierungs- oder/und Festphasenbindungsgruppen bzw. deren Konjugate können in einem immunologischen Nachweisverfahren oder in einem Nukleinsäure-Hybridisierungsverfahren eingesetzt werden. Dabei kann eine Verbesserung der Löslichkeit sowie eine Verringerung bzw. Verhinderung eines Signalquenching aufgrund der Bildung von Dimeren oder höheren Aggregaten erreicht werden.

Ein besonderer Vorteil dieses Verfahrens ist, daß Peptide oder Peptidderivate verwendet werden können, die mindestens eine freie Säurefunktion, insbesondere eine Carbonsäurefunktion aufweisen. Im Gegensatz zu bekannten Verfahren ist eine Blockierung der Säurefunktion nicht erforderlich.

Neben Peptiden kann man auch solche Peptidderivate aktivieren, die mindestens eine Markierungs- oder/und Festphasenbindungsgruppe, z.B. einen Metallkomplex, eine Fluoreszenzgruppe oder eine Biotingruppe tragen.

Die Aktivierungsreaktion wird vorzugsweise in einem organischen Lösungsmittel, z.B. Dimethylformamid, in Gegenwart einer Base, z.B. einem tertiären Amin wie etwa Triethylamin, durchgeführt. Der Korksäure-bis-N-hydroxysuccinimidester wird vorzugsweise in einem molaren Überschuß von 2:1 bis 10:1 bezüglich des Peptids oder Peptidderivats eingesetzt.

Weiterhin wird die vorliegende Erfindung durch nachfolgende Beispiele und Abbildungen erläutert. Es zeigen:
- Abb.1-3: erfindungsgemäße Metallkomplexe,
- Abb. 4: ein zum Aufbau eines erfindungsgemäßen Metallkomplexes geeignetes Aminosäure-Metallkomplex-Konjugat, und
- Abb. 5: ein aktiviertes Peptid-Metallkomplex-Konjugat.

### Beispiel 1

### Herstellung eines Metallkomplexes mit geladenem Linker

6 mmol des Metallkomplexes Ru(Bipyridin)₂-(Bipyridin-C0-N-Hydroxysuccinimidester) gemäß EP-A-0 580 979 werden in 50 ml Dimethylformamid gelöst und dazu eine Lösung von Fmoc-Lysin in Dimethylformamid getropft. Das Lösungsmittel wird im Hochvakuum entfernt. Der Rückstand wird in wenig Aceton gelöst, mit 300 ml Chloroform versetzt und kurz zum Sieden erhitzt. Man läßt abkühlen und trennt das abgeschiedene Öl vom Lösungsmittel ab. Durch Trocknen erhält man die gewünschte Verbindung Ru(bpy)₂(bpy-C0-(Fmoc)-Lys) als Festsubstanz. Die Abspaltung der Fmoc-Schutzgruppe erfolgt durch Umsetzung in Dioxan/Aceton mit einem 4-fachen Überschuß an Piperidin für 2 stunden bei 80°C. Nach dem Abkühlen trennt man den öligen Rückstand ab und führt eine Chloroform/Wasser-Extraktion durch. Die wäßrige Phase wird isoliert und man erhält die Verbindung Ru(bpy)₂(bpy-C0-Lys) als roten Feststoff.

60 mg dieser Verbindung werden in 10 ml Aceton gelöst, Maleimidopropionsäure-N-Hydroxysuccinimidester zugefügt und 4 Stunden bei Raumtemperatur gerührt. Der Rückstand wird über präparative HPLC gereinigt. Man erhält 17 mg der Verbindung Ru(bpy)₂(bpy-C0-Lys-MP). Diese Verbindung ist in Abb. 1 dargestellt.

### Beispiel 2

### Herstellung eines Metallkomplexes mit geladenem Linker

Ru(bpy)₂(bpy-C0-Lys) gemäß Beispiel 1 und der 10-fache molare Überschuß an Korksäure-bis-N-hydroxysuccinimidester werden in Dimethylformamid gelöst und 2 h bei Raumtemperatur gerührt. Das Lösungsmittel wird im Hochvakuum entfernt, der Rückstand mit Wasser extrahiert und nach Hexanbehandlung lyophilisiert. Die Reinigung erfolgt über HPLC (100% H₂0, in 20 min. auf 100% Acetonitril, C₁₈, 3 µm-Säule, Flußrate: 1 ml/min., Retentionszeit: 10,00 min.). Die erhaltene Verbindung ist in Abb. 2 dargestellt.

### Beispiel 3

### Herstellung von Metallkomplexen über eine Festphasen-Peptidsynthese

Die Metallkomplexe mit geladenem Linker wurden mittels Fluorenylmethyloxycarbonyl-(Fmoc)-Festphasenpeptidsynthese an einem Batch-Peptidsynthesizer, z.B. von Applied Biosystems A431 oder A433, hergestellt. Dazu wurden jeweils 4.0 Äquivalente der in Tabelle 1 dargestellten Aminosäurederivate verwendet:

**Tabelle 1**

| | |
|---|---|
| **A** | Fmoc-Ala-OH |
| C | Fmoc-Cys(Trt)-OH |
| D | Fmoc-Asp(OtBu)-OH |
| E | Fmoc-Glu(OtBu)-OH |
| F | Fmoc-Phe-OH |
| G | Fmoc-Gly-OH |
| H | Fmoc-His(Trt)-OH |
| I | Fmoc-Ile-OH |
| K1 | Fmoc-Lys(Boc)-OH |
| K2 | Boc-Lys(Fmoc)-OH |
| K3 | Fmoc-Lys(BPRu)-OH |
| L | Fmoc-Leu-OH |
| M | Fmoc-Met-OH |
| N | Fmoc-Asn(Trt)-OH |
| P | Fmoc-Pro-OH |
| Q | Fmoc-Gln(Trt)-OH |
| R | Fmoc-Arg(Pmc)-OH |
| S | Fmoc-Ser(tBu)-OH |
| T | Fmoc-Thr(tBu)-OH |
| U | Fmoc-βAlanin-OH |
| V | Fmoc-Val-OH |
| W | Fmoc-Trp-OH |
| Y | Fmoc-Tyr(tBu)-OH |
| Z | Fmoc-ε-Aminocapronsäure-OH |
| Nle | Fmoc-ε-Norleucin-OH |
| Abu | Fmoc-γ-Aminobuttersäure-OH |

Bei der Variante (a) - Einführung des Metallkomplexes nach Beendigung der Festphasensynthese - wurde ein aktivierter Ruthenium(bipyridyl)₃-Komplex (BPRu), z.B. Ru(bpy)₂(bpy-C0-NHS) (vgl. Beispiel 1) an die N-terminale Aminosäure des Peptids gekoppelt.

Gemäß Variante (b) erfolgte die Einführung von Metallchelatgruppen in die Peptidsequenz durch direkten Einbau von Metallchelat-gekoppelten Aminosäurederivaten, z.B. am Carboxylterminus der Sequenz über einen mit Metallchelat-Aktivester ε-derivatisierten Lysinrest, z.B. das Lysin-Derivat K3 (Abb. 4)

Die Aminosäuren oder Aminosäurederivate wurden in N-Methylpyrrolidon gelöst. Das Peptid wurde an 400-500 mg 4-(2',4'-Dimethoxyphenyl-Fmoc-Aminomethyl)-Phenoxy-Harz (Tetrahedron Letters 28 (1987), 2107) mit einer Beladung von 0,4-0,7 mmol/g aufgebaut (JACS 95 (1973), 1328). Die Kupplungsreaktionen wurden bezüglich des Fmoc-Aminosäurederivats mit 4 Äquivalenten Dicyclohexylcarbodiimid und 4 Äquivalenten N-Hydroxybenzotriazol in Dimethylformamid als Reaktionsmedium während 20 min durchgeführt. Nach jedem Syntheseschritt wurde die Fmoc-Gruppe mit 20%igem Piperidin in Dimethylformamid in 20 min abgespalten.

Bei Anwesenheit von Cysteinresten in der Peptidsequenz erfolgte unmittelbar nach Beendigung der Synthese eine Oxidation an der Festphase mit Jod in Hexafluorisopropanol/Dichlormethan.

Die Freisetzung des Peptids vom Träger und die Abspaltung der säurelabilen Schutzgruppen erfolgte mit 20 ml Trifluoressigsäure, 0,5 ml Ethandithiol, 1 ml Thioanisol, 1,5 g Phenol und 1 ml Wasser in 40 min bei Raumtemperatur. Die Reaktionslösung wurde anschließend mit 300 ml gekühltem Diisopropylether versetzt und zur vollständigen Fällung des Peptids 40 min bei 0°C gehalten. Der Niederschlag wurde abfiltriert, mit Diisopropylether nachgewaschen, mit wenig 50 %-iger Essigsäure gelöst und lyophilisiert. Das erhaltene Rohmaterial wurde mittels präparativer HPLC an Delta-PAK RP C18-Material (Säule 50 x 300 mm, 100 Å, 15 µ) über einen entsprechenden Gradienten (Eluent A: Wasser, 0,1% Trifluoressigsäure, Eluent B: Acetonitril, 0,1% Trifluoressigsäure) in ca. 120 min. aufgereinigt. Die Identität des eluierten Materials wurde mittels Ionenspray-Massenspektrometrie geprüft.

Die Einführung der Metallchelat-Markierung erfolgte gemäß Variante (a) über entsprechende Aktivester-Derivate an die freie N-terminale Aminogruppe des trägergebundenen Peptids. Hierzu wurden 4 Äquivalente Ruthenium(bipyridyl)₃-Komplex (BPRu) pro freie primäre Aminofunktion, aktiviert mit N-Hydroxybenzotriazol/Dicyclohexylcarbodiimid und in wenig DMSO gelöst, zugetropft und bei Raumtemperatur gerührt. Der Umsatz wurde über analytische HPLC verfolgt. Nach Abspaltung vom Träger wurde das Produkt mittels präparativer HPLC aufgereinigt. Die Identität des eluierten Materials wurde mittels Ionenspray-Massenspektrometrie geprüft.

Die Herstellung der Peptide erfolgte auch durch eine Kombination von Variante (a) und (b), d.h. Einbau von Metallchelat-gekoppelten Aminosäurederivaten innerhalb der Sequenz, Abspaltung der N-terminalen Fmoc-Gruppe und Reaktion der freien N-terminalen Aminogruppe mit einem Metallchelat-Aktivesterderivat.

Bei einem ausschließlich direkten Einbau der Metallchelatgekoppelten Aminosäurederivate während der Festphasensynthese gemäß Variante (b) war eine nachträgliche Einführung von Metallchelat-Aktivestern nicht mehr erforderlich.

Ein Beispiel für einen durch Festphasensynthese hergestellten Metallkomplex ist in Abb. 3 gezeigt. Zur Einführung der Maleinimidfunktion wurde das Peptid in 0,1 M Kaliumphosphatpuffer pH 7,0 gelöst und mit einem Äquivalent Maleinimidpropionsäure-N-hydroxysuccinimidester in DMSO versetzt und 16 h bei 25 °C gerührt. Der Ansatz wurde über präparative HPLC (siehe oben) aufgereinigt. Die Identität des eluierten Materials wurde mittels Ionenspray-Massenspektrometrie geprüft. Die Einführung einer reaktiven-N-Hydroxysuccinimidesterfunktion erfolgte entsprechend DE-A-43 02 241.

### Beispiel 4

### Anwendung von Metallkomplexen mit geladenem Linker in immunologischen Tests

Es wurde ein Doppel-Antigen-Brückentest zum Nachweis spezifischer Antikörper gegen Hepatitis C-Virus (HCV) durchgeführt. Hierbei wurde die Probeflüssigkeit mit einem Ruthenium-markierten Antigen und einem biotinylierten Antigen gegen den zu bestimmenden Antikörper in Gegenwart einer Streptavidin-beschichteten Festphase inkubiert. Das Vorhandensein von Anti-HCV-Antikörpern in der Probeflüssigkeit wurde durch Bestimmung der Markierung in der Festphase durch Elektrochemilumineszenz nach dem Flash-System bestimmt.

Als Antigen wurde ein HCV-Polypeptid, welches die Aminosäuren 1207-1488 von HCV enthält, verwendet. Die Aminosäuresequenz und Herstellung eines derartigen Polypeptids ist in DE-A-44 28 705.4 beschrieben.

Zur Derivatisierung des HCV-Polypeptids mit Succinimidesteraktivierten Rutheniumkomplexen wurde das Polypeptid in einem 100 mM Natriumphosphatpuffer pH 6,5, 0,1 % SDS in einer Proteinkonzentration von 10 mg/ml gelöst. Durch Zusatz von 5 M NaOH wurde der pH-Wert auf 8,5 eingestellt und die Lösung mit Dithiothreitol auf eine Endkonzentration von 2 mM aufgestockt. Zu dieser Lösung wurde die der gewünschten Angebotsstöchiometrie entsprechende Menge eines Succinimidesteraktivierten Rutheniumkomplexes in DMSO zugegeben und anschließend für 60 min bei 65 °C unter Rühren inkubiert. Die Reaktion wurde durch Aufstocken des Reaktionsgemisches mit Lysin auf eine Endkonzentration von 10 mM und eine weitere Inkubation für 30 min abgestoppt. Anschließend wurde der Ansatz gegen 100 mM Natriumphosphatpuffer pH 6,5, 0,1 % SDS dialysiert. Die resultierende Proteinlösung wurde mit Saccharose (Endkonzentration 6,5 % (w/v)) versetzt und in Portionen lyophilisiert.

Zur Herstellung eines mit einem Maleinimid-aktivierten Rutheniumkomplex derivatisierten HCV-Polypeptids wurde das Polypeptid in 100 mM Natriumphosphatpuffer pH 6,5, 0,1 % SDS (Proteinkonzentration 10 mg/ml) aufgenommen. Zu dieser Lösung wurde eine der gewünschten Angebotsstöchiometrie entsprechende Menge des Maleinimid-aktivierten Rutheniumkomplexes in DMSO zugegeben und 60 min bie 25 °C unter Rühren inkubiert. Die Reaktion wurde durch Aufstocken des Reaktionsgemisches mit Cystein auf eine Endkonzentration von 10 mM und weitere Inkubation für 30 min abgestoppt. Das Reaktionsgemisch wurde daraufhin wie oben beschrieben dialysiert, mit Saccharose versetzt und in Portionen lyophilisiert.

Es wurden 3 Experimente durchgeführt, in denen jeweils unterschiedliche ruthenylierte Antigene eingesetzt wurden. Für Experiment A (Vergleich) wurde das Polypeptid an den in den Beispielen 1 und 2 als Ausgangsmaterial verwendeten Ruthenium-Komplex gemäß EP-A-0 580 979 in einem stöchiometrischen Verhältnis von 1:3 gekoppelt. Für Experiment B wurde das Polypeptid mit dem in Beispiel 1 hergestellten erfindungsgemäßen Ruthenium-Komplex (Abb. 1) in einem stöchiometrischen Verhältnis von 1:1 gekoppelt. Für Experiment C wurde das Polypeptid mit dem in Beispiel 3 hergestellten Ruthenium-Komplex (Abb. 3) im stöchiometrischen Verhältnis von 1:1 gekoppelt. Als biotinyliertes Antigen wurde in allen 3 Experimenten ein Polypeptid verwendet, das im stöchiometrischen Verhältnis von 1:6 an ein Maleinimid-aktiviertes Biotin gekoppelt worden war. Das ruthenylierte und das biotinylierte Antigen wurden jeweils in einer Konzentration von 400 ng/ml Testflüssigkeit eingesetzt.

In Tabelle 4 ist das Ergebnis der Experimente A, B und C in ECL-Counts dargestellt. Es ist ersichtlich, daß erst die Verwendung der erfindungsgemäßen Metallkomplexe mit geladenem Linker als Markierungsgruppen eine zuverlässige Unterscheidung zwischen einer negativen Serumprobe und einer kritischen positiven Serumprobe erlaubt. Dies zeigt sich in einem höheren Verhältnis positiv/negativ.

**Tabelle 2**

| Experiment | A (Vergleich) | B | C |
|---|---|---|---|
| negative Probe | 323317 | 132288 | 14467 |
| positive Probe | 465769 | 1323338 | 319752 |
| Verhältnis positiv/negativ | 1,4 | 10 | 22 |

### Beispiel 5

### Anwendung von Metallkomplexen und Biotingruppen mit geladenem Linker in immunologischen Tests

Es wurde ein Doppel-Antigen-Brückentest zum Nachweis spezifischer Antikörper gegen HIV I entsprechend dem Protokoll gemäß Beispiel 4 durchgeführt.

Als Antigen wurde das HIV-Polypeptid gp32 verwendet.

Der Einbau der Peptidlinkersequenzen "EEE" und "EEEUZU" zwischen dem Antigen und dem Rutheniumkomplex oder/und Biotin führte zu einer deutlichen Absenkung des unspezifischen Signals im Vergleich zu den Antigenen ohne Linkersequenz, während das spezifische Signal bei HIV-positiven Proben im wesentlichen erhalten blieb. Durch Verwendung von Markierungs- oder/und Festphasenbindungsgruppen mit geladenen Linkern kann somit eine wesentlich bessere Dynamik bei der Bestimmung von Analyten erzielt werden.

### Beispiel 6

### Einführung von N-Hydroxysuccinimidestergruppen in Peptidderivate

250 mg Korksäure-bis-N-Hydroxysuccinimidester (DSS) wurden zusammen mit 50 µl Triethylamin in Dimethylformamid gelöst. Dazu wurde eine Lösung des Peptidderivats BPRu-UEEK (100 mg in DMF), das gemäß der in Beispiel 3 beschriebenen Standardmethode hergestellt wurde, zugetropft. Nach ca. 15 min wurde das DMF im Hochvakuum entfernt, der Rückstand in Wasser aufgenommen und das ungelöste DSS abfiltriert. Das Filtrat wurde lyophilisiert.

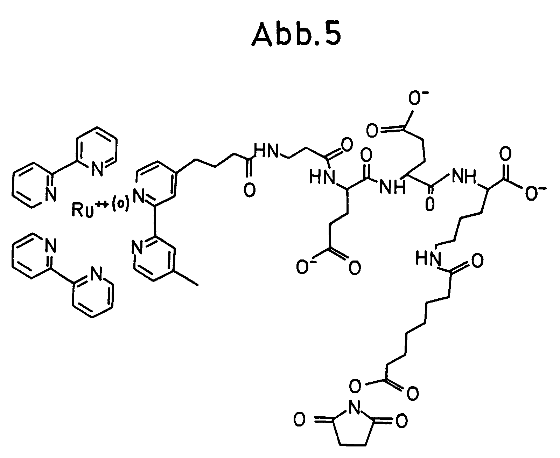

Es wurde das in Abb. 5 dargestellte Produkt erhalten. Die Reinheit gemäß HPLC war 91 %. Die Analytik durch NMR und MS entsprach dem erwarteten Produkt.

Auf analoge Weise wurde ein zweifach ruthenyliertes Peptidderivat mit der Sequenz Ac-K (BPRu) UEUEUK-(DSS)-UEUEUK (BPRu) UE hergestellt. Statt ruthenylierter Peptide lassen sich auf analoge Weise auch biotinylierte oder mit anderen Markierungsgruppen versehene bzw. unmarkierte Peptide mit DSS aktivieren. Überraschenderweise verläuft die Reaktion in Gegenwart freier Carbonsäurefunktionen glatt.

## Patentansprüche

1. Metallkomplexe mit der allgemeinen Formel (I):
[M(L₁L₂L₃)]ₙ - Xₘ A (I)
worin
- M ein zwei- oder dreiwertiges Metallkation ausgewählt aus Seltenerde- oder Übergangmetallionen ist,
- L₁, L₂ und L₃ gleich oder verschieden sind und Liganden mit mindestens zwei stickstoffhaltigen Heterocyclen bedeuten, wobei L₁, L₂ und L₃ über Stickstoffatome an das Metallkation gebunden sind,
- X eine reaktive funktionelle Gruppe ist, die an mindestens einen der Liganden L₁, L₂, L₃ kovalent über einen Linker gebunden ist,
- n eine ganze Zahl von 1 bis 10 ist,
- m eine ganze Zahl von 1 bis 6 ist und
- A die gegebenenfalls zum Ladungsausgleich erforderlichen Gegenionen bedeutet,
dadurch gekennzeichnet,
daß der Linker mindestens einen positiven oder/und negativen Ladungsträger enthält.

2. Komplex nach Anspruch 1, dadurch gekennzeichnet, daß das Metallkation M ein Ruthenium-, Rhenium-, Osmium-, Chrom- oder Iridiumion ist.

3. Komplex nach Anspruch 1 oder 2, dadurch gekennzeichnet, daß das Metallkation M ein Rutheniumion ist.

4. Komplex nach einem der Ansprüche 1 bis 3, dadurch gekennzeichnet, daß die Liganden L₁, L₂, L₃ Bipyridin-oder/und Phenanthrolin-Ringsysteme enthalten.

5. Komplex nach einem der Ansprüche 1 bis 4, dadurch gekennzeichnet, daß die reaktive funktionelle Gruppe X ein Carbonsäurehalogenid, ein Carbonsäureanhydrid, Aktivester, ein Maleimid oder eine photoaktivierbare Gruppe ist.

6. Komplex nach einem der Ansprüche 1 bis 5, dadurch gekennzeichnet, daß die Gegenionen A Hexafluorophos-phat-, Trifluoracetat- oder Tetrafluoroborat-Gruppen sind.

7. Komplex nach einem der Ansprüche 1-6,
**dadurch gekennzeichnet**, daß der Linker mindestens einen negativen Ladungsträger, ausgewählt aus Phosphat-, Phosphonat-, Sulfonat- und Carboxylatgruppen enthält.

8. Komplex nach Anspruch 7,
**dadurch gekennzeichnet**,
daß der Linker mindestens eine Carboxylatgruppe enthält.

9. Komplex nach einem der Ansprüche 1-6,
**dadurch gekennzeichnet**,
daß der Linker mindestens einen positiven Ladungsträger, ausgewählt aus Amino- und substituierten Aminogruppen enthält.

10. Komplex nach Anspruch 9,
**dadurch gekennzeichnet**,
daß die Amino- und substituierten Aminogruppen als Elektronendonor für den Metallkomplex dienen können.

11. Komplex nach einem der Ansprüche 1-10,
**dadurch gekennzeichnet**,
daß der Linker bis zu 10 Ladungsträger enthält.

12. Komplex nach Anspruch 11,
**dadurch gekennzeichnet**,
daß der Linker 2-8 Ladungsträger enthält.

13. Komplex nach einem der Ansprüche 1-12,
**dadurch gekennzeichnet**,
daß der Linker zumindest teilweise aus Aminocarbonsäure-Einheiten aufgebaut ist, die über Peptidbindungen miteinander verknüpft sind.

14. Komplex nach Anspruch 13,
**dadurch gekennzeichnet**,
daß die Ladungsträger von polyfunktionellen Aminocarbonsäuren stammen, die nach Einbau in den Linker noch mindestens einen freien Ladungsträger enthalten.

15. Komplex nach Anspruch 14,
**dadurch gekennzeichnet**,
daß die Ladungsträger von trifunktionellen Aminocarbonsäuren stammen, die
(a) eine Amninogruppe und zwei Carboxylatgruppen oder
(b) zwei Aminogruppen und eine Carboxylatgruppe enthalten.

16. Komplex nach Anspruch 15,
**dadurch gekennzeichnet**,
daß die trifunktionellen Aminocarbonsäuren ausgewählt sind aus Lysin, Ornithin, Hydroxylysin, Asparaginsäure und Glutaminsäure.

17. Komplex nach einem der Ansprüche 1 bis 16 mit der allgemeinen Formel (III): 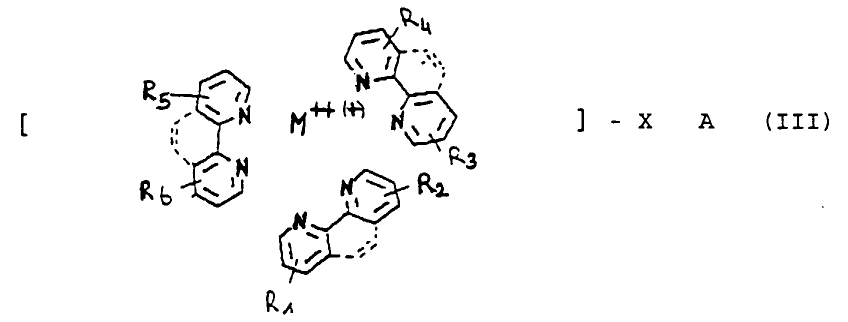 worin M, X und A wie in Anspruch 1 definiert sind, R₁, R₂, R₃, R₄, R₅ und R₆ gleich oder verschieden sind und jeweils einen oder mehrere Substituenten bedeuten, unter der Voraussetzung, daß X über einen der Substituenten R₁, R₂, R₃, R₄, R₅ oder R₆ als Linker an einen der Liganden gebunden ist.

18. Komplex nach Anspruch 17 mit der allgemeinen Formel (IIIa): 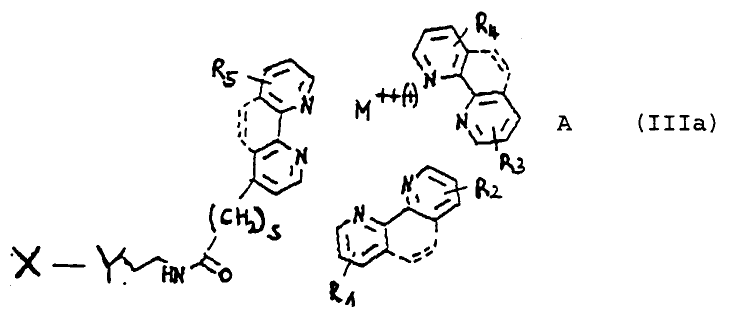 worin M, X und A wie in Anspruch 1 definiert sind, R₁, R₂, R₃, R₄ und R₅ wie in Anspruch 16 definiert sind, s eine ganze Zahl von 0 bis 6 ist und Y den Linker bedeutet.

19. Komplex nach einem der Ansprüche 1 bis 16 mit der allgemeinen Formel (IV): 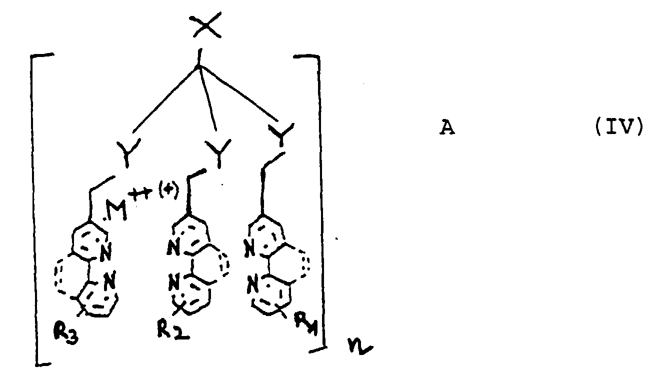 worin M, X, n und A wie in Anspruch 1 definiert sind, R₁, R₂ und R₃ gleich oder verschieden sind und jeweils einen oder mehrere Substituenten bedeuten und Y jeweils einen Linker mit mindestens einem Ladungsträger bedeutet.

20. Konjugat, umfassend eine biologische Substanz, an die mindestens ein Metallkomplex nach einem der Ansprüche 1 bis 19 gekoppelt ist.

21. Konjugat nach Anspruch 20, dadurch gekennzeichnet, daß die biologische Substanz Biotin, ein Antikörper oder Antikörperfragment, eine Nukleinsäure, ein Polypeptidantigen, ein immunologisch reaktives Peptid oder ein Hapten ist.

22. Verwendung von Metallkomplexen nach einem der Ansprüche 1 bis 19 oder Konjugaten nach Anspruch 20 oder 21 in einem immunologischen Nachweisverfahren oder in einem Nukleinsäure-Hybridisierungsverfahren.

23. Verwendung nach Anspruch 22 in einem Lumineszenzverfahren.

24. Verwendung nach Anspruch 22 oder 23 in einem Elektrochemilumineszenzverfahren.

## Claims

1. Metal complexes of the general formula (I):
[M(L₁L₂L₃)]ₙ - Xₘ A (I)
in which
- M is a divalent or trivalent metal cation selected from rare earth or transition metal ions,
- L₁, L₂ and L₃ are the same or different and denote ligands with at least two nitrogen-containing heterocycles wherein L₁, L₂ and L₃ are bound to the metal cation via nitrogen atoms,
- X is a reactive functional group which is covalently bound via a linker to at least one of the ligands L₁, L₂ and L₃,
- n is an integer from 1 to 10,
- m is an integer from 1 to 6 and
- A denotes the counterions which may be required to balance the charge
**wherein**
the linker contains at least one positive or/and negative charge carrier.

2. Complex as claimed in claim 1, **wherein** the metal cation M is a ruthenium ion, rhenium ion, osmium ion, chromium ion or iridium ion.

3. Complex as claimed in claim 1 or 2, **wherein** the metal cation M is a ruthenium ion.

4. Complex as claimed in one of the claims 1 to 3, **wherein** the ligands L₁, L₂ and L₃ contain bipyridine or/and phenanthroline ring systems.

5. Complex as claimed in one of the claims 1 to 4, **wherein** the reactive functional group X is a carboxylic acid halogenide, carboxylic acid anhydride, active ester, a maleimide or a group that can be photo-activated.

6. Complex as claimed in one of the claims 1 to 5, **wherein** the counterions A are hexafluorophosphate, trifluoroacetate or tetrafluoroborate groups.

7. Complex as claimed in one of the claims 1-6, **wherein** the linker contains at least one negative charge carrier selected from phosphate, phosphonate, sulfonate and carboxylate groups.

8. Complex as claimed in claim 7,
**wherein** the linker contains at least one carboxylate group.

9. Complex as claimed in one of the claims 1-6, **wherein** the linker contains at least one positive charge carrier selected from amino groups and substituted amino groups.

10. Complex as claimed in claim 9,
**wherein** the amino groups and substituted amino groups can serve as the electron donor for the metal complex.

11. Complex as claimed in one of the claims 1-10,
**wherein** the linker contains up to 10 charge carriers.

12. Complex as claimed in claim 11,
**wherein** the linker contains 2-8 charge carriers.

13. Complex as claimed in one of the claims 1-12, **wherein** the linker is at least partially composed of aminocarboxylic acid units which are linked together via peptide bonds.

14. Complex as claimed in claim 13,
**wherein** the charge carriers are derived from polyfunctional aminocarboxylic acids that still contain at least one free charge carrier after incorporation into the linker.

15. Complex as claimed in claim 14,
**wherein** the charge carriers are derived from trifunctional aminocarboxylic acids which
(a) contain one amino group and two carboxylate groups or
(b) two amino groups and one carboxylate group.

16. Complex as claimed in claim 15,
**wherein** the trifunctional aminocarboxylic acids are selected from lysine, ornithine, hydroxylysine, aspartic acid and glutamic acid.

17. Complex as claimed in one of the claims 1 to 16 of the general formula (III): 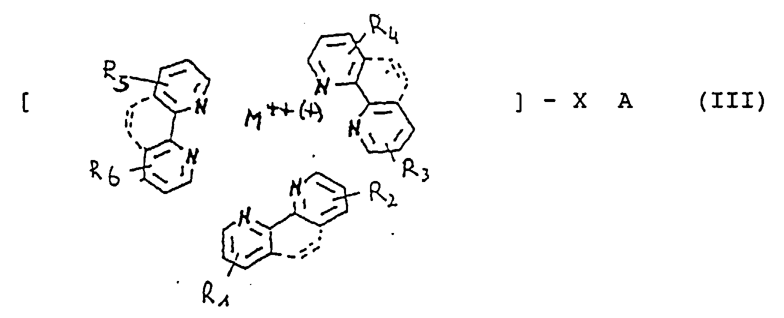 in which M, X and A are defined as in claim 1, R₁, R₂, R₃, R₄, R₅ and R₆ are the same or different and each denote one or several substituents provided that X is bound to one of the ligands via one of the substituents R₁, R₂, R₃, R₄, R₅ and R₆ as the linker.

18. Complex as claimed in claim 17 having the general formula (IIIa) 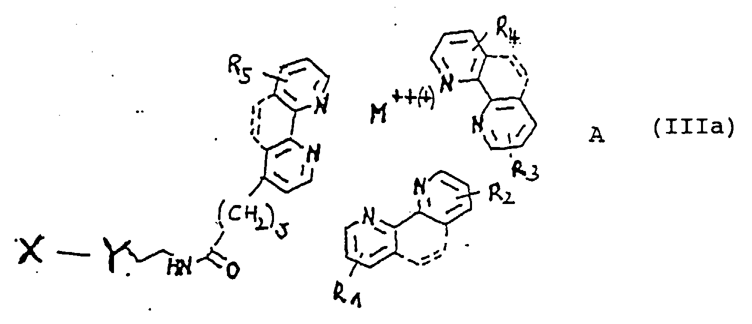 in which M, X and A are defined as in claim 1, R₁, R₂, R₃, R₄ and R₅ are defined as in claim 16, s is an integer from 0 to 6 and Y denotes the linker.

19. Complex as claimed in one of the claims 1 to 16 having the general formula (IV): 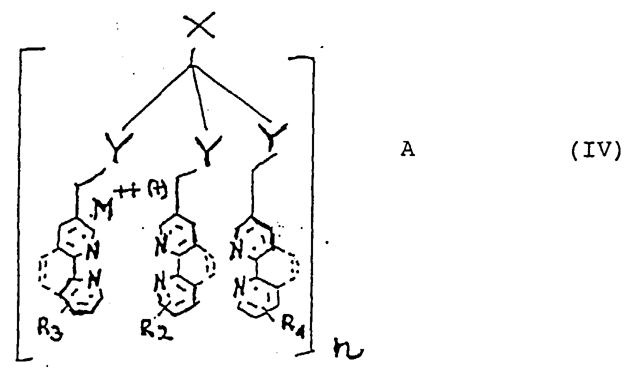 in which M, X, n and A are defined as in claim 1, R₁, R₂ and R₃ are the same or different and each denotes one or several substituents and Y in each case denotes a linker with at least one charge carrier.

20. Conjugate, comprising a biological substance to which at least one metal complex is coupled as claimed in one of the claims 1 to 19.

21. Conjugate as claimed in claim 20,
**wherein** the biological substance is biotin, an antibody or antibody fragment, a nucleic acid, a polypeptide antigen, an immunological reactive peptide or a hapten.

22. Use of metal complexes as claimed in one of the claims 1 to 19 or conjugates as claimed in claim 20 or 21 in an immunological detection method or in a nucleic acid hybridization method.

23. Use as claimed in claim 22 in a luminescence method.

24. Use as claimed in claim 22 or 23 in a electrochemiluminescence method.

## Revendications

1. Complexes métalliques de formule générale (I):
[M(L₁L₂L₃)]ₙ - Xₘ A (I)
où
- M est un cation métallique di- ou trivalent choisi parmi les ions des métaux des terres rares ou de transition,
- L₁, L₂ et L₃ sont identiques ou différents et représentent des ligands ayant au moins deux hétérocycles azotés, où L₁, L₂ et L₃ sont liés au cation métallique par des atomes d'azote,
- X est un groupe fonctionnel réactif qui est lié de manière covalente à au moins l'un des ligands L₁, L₂, L₃ par le biais d'un lieur,
- n est un nombre entier de 1 à 10,
- m est un nombre entier de 1 à 6 et
- A représente les contre-ions éventuellement nécessaires pour compenser les charges,
caractérisés en ce que le lieur contient au moins un porteur de charge(s) positif et/ou négatif.

2. Complexe selon la revendication 1 caractérisé en ce que le cation métallique M est un ion ruthénium, rhénium, osmium, chrome ou iridium.

3. Complexe selon la revendication 1 ou 2 caractérisé en ce que le cation métallique M est un ion ruthénium.

4. Complexe selon l'une des revendications 1 à 3 caractérisé en ce que les ligands L₁, L₂, L₃ contiennent des systèmes cycliques bipyridine et/ou phénanthroline.

5. Complexe selon l'une des revendications 1 à 4 caractérisé en ce que le groupe fonctionnel réactif X est un halogénure d'acide carboxylique, un anhydride d'acide carboxylique, un ester actif, un maléimide ou un groupe photo-activable.

6. Complexe selon l'une des revendications 1 à 5 caractérisé en ce que les contre-ions A sont des groupes hexafluorophosphate, trifluoroacétate ou tétrafluoroborate.

7. Complexe selon l'une des revendications 1-6 caractérisé en ce que le lieur contient au moins un porteur de charge(s) négatif, choisi parmi les groupes phosphate, phosphonate, sulfonate et carboxylate.

8. Complexe selon la revendication 7 caractérisé en ce que le lieur contient au moins un groupe carboxylate.

9. Complexe selon l'une des revendications 1-6 caractérisé en ce que le lieur contient au moins un porteur de charge(s) positif choisi parmi les groupes amino et amino substitués.

10. Complexe selon la revendication 9 caractérisé en ce que les groupes amino et amino substitués peuvent servir de donneur d'électrons pour le complexe métallique.

11. Complexe selon l'une des revendications 1-10 caractérisé en ce que le lieur contient jusqu'à 10 porteurs de charge(s).

12. Complexe selon la revendication 11 caractérisé en ce que le lieur contient 2-8 porteurs de charge(s).

13. Complexe selon l'une des revendications 1-12 caractérisé en ce que le lieur est constitué au moins en partie par des unités d'acides aminocarboxyliques qui sont liées entre elles par des liaisons peptidiques.

14. Complexe selon la revendication 13 caractérisé en ce que les porteurs de charge(s) sont issus d'acides aminocarboxyliques polyfonctionnels qui contiennent encore au moins un porteur de charge(s) libre après incorporation dans le lieur.

15. Complexe selon la revendication 14 caractérisé en ce que les porteurs de charge(s) sont issus d'acides aminocarboxyliques trifonctionnels qui contiennent
(a) un groupe amino et deux groupes carboxylate ou
(b) deux groupes amino et un groupe carboxylate.

16. Complexe selon la revendication 15 caractérisé en ce que les acides aminocarboxyliques trifonctionnels sont choisis parmi la lysine, l'ornithine, l'hydroxylysine, l'acide aspartique et l'acide glutamique.

17. Complexe selon l'une des revendications 1 à 16 de formule générale (III): 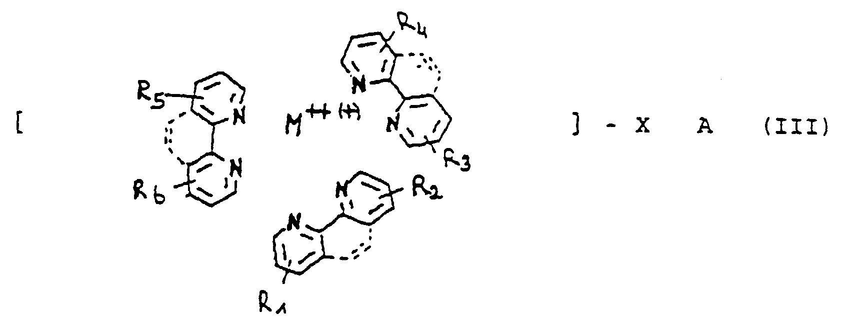 où M, X et A sont définis comme dans la revendication 1, R₁, R₂, R₃, R₄, R₅ et R₆ sont identiques ou différents et représentent chacun un ou plusieurs substituants, à condition que X soit lié à l'un des ligands par le biais de l'un des substituants R₁, R₂, R₃, R₄, R₅ et R₆ comme lieur.

18. Complexe selon la revendication 17 de formule générale (IIIa): 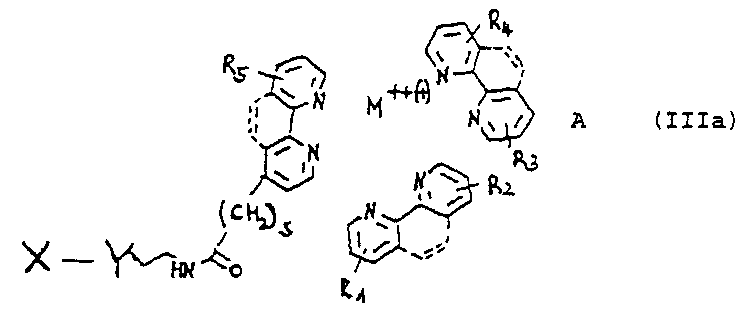 où M, X et A sont définis comme dans la revendication 1, R₁, R₂, R₃, R₄ et R₅ sont définis comme dans la revendication 16, s est un nombre entier de 0 à 6 et Y représente le lieur.

19. Complexe selon l'une des revendications 1 à 16 de formule générale (IV): 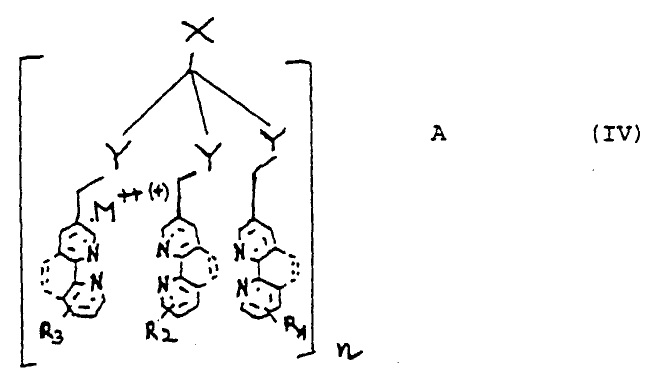 où M, X, n et A sont définis comme dans la revendication 1, R₁, R₂ et R₃ sont identiques ou différents et représentent chacun un ou plusieurs substituants et Y représente dans chaque cas un lieur ayant au moins un porteur de charge(s).

20. Conjugué comprenant une substance biologique à laquelle est couplé au moins un complexe métallique selon l'une des revendications 1 à 19.

21. Conjugué selon la revendication 20 caractérisé en ce que la substance biologique est la biotine, un anticorps ou fragment d'anticorps, un acide nucléique, un antigène polypeptidique, un peptide immunologiquement réactif ou un haptène.

22. Utilisation de complexes métalliques selon l'une des revendications 1 à 19 ou de conjugués selon la revendication 20 ou 21 dans un procédé de mise en évidence immunologique ou dans un procédé faisant intervenir l'hybridation d'acides nucléiques.

23. Utilisation selon la revendication 22 dans un procédé faisant intervenir la luminescence.

24. Utilisation selon la revendication 22 ou 23 dans un procédé faisant intervenir l'électrochimiluminescence.
